(19) Europäisches Patentamt
European Patent Office
Office européen des brevets

(11) **EP 2 621 426 B1**

(12) **EUROPÄISCHE PATENTSCHRIFT**

(45) Veröffentlichungstag und Bekanntmachung des Hinweises auf die Patenterteilung:
**13.08.2014 Patentblatt 2014/33**

(21) Anmeldenummer: **11805753.8**

(22) Anmeldetag: **24.09.2011**

(51) Int Cl.:
***A61F 9/007*** *(2006.01)*

(86) Internationale Anmeldenummer:
**PCT/DE2011/001778**

(87) Internationale Veröffentlichungsnummer:
**WO 2012/041291 (05.04.2012 Gazette 2012/14)**

(54) **STEUERUNGSVORRICHTUNG FÜR EIN OPHTHALMOCHIRURGISCHES SYSTEM**

CONTROL DEVICE FOR AN OPHTHALMOSURGICAL SYSTEM

DISPOSITIF DE COMMANDE CONÇU POUR UN SYSTÈME DE CHIRURGIE OPHTALMOLOGIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.09.2010 DE 102010047010**

(43) Veröffentlichungstag der Anmeldung:
**07.08.2013 Patentblatt 2013/32**

(73) Patentinhaber: **Carl Zeiss Meditec AG**
**07745 Jena (DE)**

(72) Erfinder: **HAUGER, Christoph**
**73431 Aalen (DE)**

(74) Vertreter: **Carl Zeiss AG - Patentabteilung**
**Carl Zeiss AG**
**C/o Patentabteilung**
**Carl-Zeiss-Straße 22**
**73447 Oberkochen (DE)**

(56) Entgegenhaltungen:
**WO-A1-2009/076717      US-A- 4 841 984**
**US-A1- 2008 319 374**

**Beschreibung**

Die Erfindung betrifft eine Steuerungsvorrichtung für ein ophthalmochirurgisches System zur Steuerung von Druck oder Volumenstrom in einer Irrigationsleitung oder einer Aspirationsleitung sowie ein ophthalmochirurgisches System mit einer solchen Steuerungsvorrichtung.

Zur Behandlung einer Augenlinsentrübung, welche in der Medizin als Grauer Star bezeichnet wird, gibt es mehrere chirurgische Techniken. Die am weitesten verbreitete Technik ist die Phakoemulsifikation, bei der eine dünne Nadel in die erkrankte Linse eingeführt und mit Ultraschall zu Schwingungen angeregt wird. Die vibrierende Nadel emulsifiziert in ihrer nächsten Umgebung die Linse derart, dass die entstehenden Linsenpartikel durch eine Leitung mittels einer Pumpe abgesaugt werden können. Dabei wird ein Spülfluid (Irrigationsfluid) zugeführt, wobei das Absaugen der Partikel und des Fluides durch eine Aspirationsleitung erfolgt. Ist die Linse vollständig emulsifiziert und entfernt worden, kann in den leeren Kapselsack eine neue künstliche Linse eingesetzt werden, so dass ein derart behandelter Patient wieder ein gutes Sehvermögen erreichen kann.

In Deutschland werden etwa 600.000 Operationen dieser Art pro Jahr durchgeführt, wobei ein solcher Eingriff eine relativ geringe Komplikationsrate mit sich bringt. Jedoch erfordert eine solche Operation immer noch viel Erfahrung vom behandelnden Chirurgen. Zwar kann die schwingende Nadel eine Augenlinse relativ zuverlässig in kleine Partikel zertrümmern, jedoch sind diese Partikel unterschiedlich groß. Ist das Partikel kleiner als der Innendurchmesser einer Aspirationsleitung, welche üblicherweise innerhalb der schwingenden Nadel verläuft, kann ein derart kleines Partikel mitsamt dem zugehörigen Fluid problemlos abgesaugt werden. Ist das Partikel aber größer als der kleinste Innendurchmesser der Aspirationsleitung, kommt es gar nicht in die Leitung hinein oder verstopft diese. Der Zustand einer verstopften Leitung wird als Okklusion bezeichnet. Die Folge einer Okklusion ist ein starker Unterdruck in der Aspirationsleitung. Zerbricht dann zum Beispiel aufgrund einer stärkeren Schwingung der Nadel das Partikel in kleinere Teile, so dass die Okklusion aufbricht, entsteht ein starker Sog im Bereich der Nadelspitze. Dabei kann es passieren, dass eine Wand eines Linsenkapselsackes an die Nadelspitze angesaugt und von der Nadel durchstochen wird. Wenn der Kapselsack durchstochen wird, führt dies zu erheblichen Komplikationen für den Patienten, welche unbedingt vermieden werden müssen. Während einer Operation bedarf es vom behandelnden Chirurgen somit höchste Aufmerksamkeit, um eine Beschädigung des Patientenauges zu vermeiden. Wenn mit so großer Vorsicht gearbeitet werden muss, dauert eine Operation aber relativ lange. Das Durchbrechen einer Okklusion wirkt sich zudem nicht nur in der Aspirationsleitung, sondern auch in der Irrigationsleitung aus. Auch dort kann es zu starken Druckschwankungen führen, wenn eine Verstopfung in der Aspirationsleitung plötzlich aufbricht.

Es ist eine Aufgabe der Erfindung, eine Steuerungsvorrichtung für ein ophthalmochirurgisches System zu schaffen, mit dem ein Druck oder ein Volumenstrom in einer Aspirationsleitung oder einer Irrigationsleitung schnell und zuverlässig gesteuert werden kann, so dass die Gefahr einer Beschädigung eines Patientenauges gering ist, wobei trotzdem die Operation schnell durchführbar ist. Es ist ferner eine Aufgabe der Erfindung, ein ophthalmochirurgisches System zu schaffen, welches mit einer solchen Steuerungsvorrichtung ausgestattet ist.

Die Aufgabe für die Steuerungsvorrichtung wird durch den Gegenstand des unabhängigen Patentanspruchs 1 gelöst. Vorteilhafte Weiterbildungen der Erfindung sind Gegenstand der Unteransprüche. Die Aufgabe für das ophthalmochirurgische System wird durch den Gegenstand des unabhängigen Patentanspruchs 7 gelöst.

Die erfindungsgemäße Steuerungsvorrichtung für ein ophthalmochirurgisches System weist auf:

- eine Messvorrichtung, mit welcher sich das Volumen einer Augenvorderkammer eines während einer Phakoemulsifikation zu behandelnden Auges zu einem ersten Zeitpunkt und zu einem zweiten Zeitpunkt ermitteln lässt, und
- eine Steuereinheit, welche in Abhängigkeit von dem ermittelten Volumen zum ersten Zeitpunkt und zum zweiten Zeitpunkt eine Steuergröße berechnet, mit der sich ein Druck oder ein Volumenstrom in einer Irrigationsleitung und/oder einer Aspirationsleitung des ophthalmochirurgischen Systems steuern lässt.

Beim Beginn einer Okklusion oder beim Aufbrechen einer Okklusion ändert sich der Druck in der Aspirationsleitung oder Irrigationsleitung. Dies kann mit einem Druckmessgerät festgestellt werden, welches mit der Irrigationsleitung oder Aspirationsleitung gekoppelt ist. Nachteilig dabei ist, dass relativ viel Zeit vergeht, bis eine Druckänderung im Auge von einem Druckmessgerät außerhalb des Auges, also zum Beispiel in einer Konsole eines ophthalmochirurgischen Systems, erfasst wird. Dies bedeutet, dass nur eine relativ träge Steuerung oder Regelung des Druckes oder Volumenstromes in der Aspirationsleitung und/oder Irrigationsleitung möglich ist, so dass Verletzungen am Patientenauge trotz großer Vorsicht des Chirurgen nicht vermieden werden können.

Die Erfindung setzt bei dem Gedanken an, die Folgen einer Druckänderung beim Beginn einer Okklusion oder beim Aufbrechen einer Okklusion direkt im Auge zu erfassen. Somit ist es nicht erforderlich, eine Änderung eines chirurgischen Parameters außerhalb des Auges zu ermitteln. Wenn eine Messvorrichtung das Volumen einer Augenvorderkammer zu einem ersten Zeitpunkt und zu einem zweiten Zeitpunkt erfasst, kann zum Beispiel eine Differenz aus dem Volumen, welches zu dem ersten Zeitpunkt besteht, und dem Volumen, welches zu dem zweiten Zeitpunkt besteht, berechnet werden. Ist der Differenzbetrag ungleich null, gibt dies indirekt eine Aussage über eine Druckänderung in der Augenvorderkammer, ohne dass ein Druck gemessen werden muss. Ein sich stark änderndes Volumen der Augenvorderkammer kann somit derart ausgewertet werden, dass chirurgische Parameter oder Steuergrößen beeinflusst werden, welche eine Verletzung eines Patientenauges verringern helfen. Der Chirurg muss somit nicht mehr mit größter Aufmerksamkeit darauf achten, die Folgen beim plötzlichen Aufbrechen einer Okklusion gering zu halten. Falls eine Okklusion auftritt, kann auf der Grundlage der Volumenmessung zum ersten Zeitpunkt und zum zweiten Zeitpunkt eine Steuereinheit des ophthalmochirurgischen Systems so gesteuert werden, dass keine so starke Druckänderung in der Aspirationsleitung beim Aufbrechen der Okklusion mehr auftritt. Da dies durch eine elektronische Steuerung schneller und effizienter geschehen kann, als durch zum Beispiel manuelle Steuerung eines Parameters mittels eines Fußpedals, kann eine Phakoemulsifikation mittels der erfindungsgemäßen Steuerungsvorrichtung in kürzerer Zeit und mit höherer Sicherheit durchgeführt werden.

Bevorzugt weist die Messvorrichtung ein optisches System wie zum Beispiel ein OCT-System zur Vermessung eines dreidimensionalen Objektbereiches auf. Ein OCT-System zum Beispiel mit einer rotierenden oder scannenden Faser ermöglicht es, einen Bereich unterhalb der Hornhaut, oberhalb der Iris und oberhalb des Linsenkapselsackes zu erfassen. Wenn sich eine starke Druckänderung ankündigt, kann dies mit einer Volumenänderung der Augenvorderkammer indirekt ermittelt werden.

Die Messgenauigkeit lässt sich erhöhen, wenn sich mittels der Messvorrichtung das Volumen der Nadel eines Phakohandstückes innerhalb der Augenvorderkammer ermitteln lässt. Während einer Operation wird die schwingende Nadel in der Augenvorderkammer mal im Randbereich der Augenlinse oder im mittleren Bereich der Augenlinse bewegt. Dabei verändert sich das in der Augenvorderkammer beanspruchte und verdrängte Volumen durch die Phakonadel. Wenn diese rein durch die Bewegung der Nadel verursachte Volumenänderung miterfasst wird, kann damit eine höhere Sicherheit erreicht werden, dass die Steuerungsvorrichtung keine gefährliche Druckänderung in der Augenvorderkammer aufgrund der Bewegung der Nadel ermittelt.

Gemäß einer Weiterbildung der Erfindung ist die Messvorrichtung geeignet, die Topographie einer Hornhaut des mittels Phakoemulsifikation zu behandelnden Auges zu vermessen. Bei einer Druckänderung ändert sich nicht nur das Volumen in der Augenvorderkammer, sondern auch zwangsweise die Geometrie und Kontur der wie eine Membran sich verhaltenden Hornhaut. Wenn sowohl die Volumenmessung der Augenvorderkammer als auch die Vermessung einer Topographie der Hornhaut durchgeführt wird, kann mit noch höherer Sicherheit eine Aussage über eine Druckänderung in der Augenvorderkammer getroffen werden.

Vorzugsweise weist die Messvorrichtung ein Keratometer auf, um die Topographie der Hornhaut zu erfassen.

Gemäß der Erfindung lässt sich mittels der Steuergröße vorzugsweise eine Pumpleistung einer Aspirationspumpe, ein Volumenstrom eines Fluids zur Belüftung der Aspirationsleitung oder ein Ventil in der Irrigationsleitung steuern. Durch die Beeinflussung der Volumenströme in der Irrigationsleitung und/oder Aspirationsleitung kann somit einer starken Druckänderung wirksam entgegen gewirkt werden.

Weitere Vorteile und Merkmale der Erfindung werden mit Bezug auf die nachfolgenden Zeichnungen erklärt, in welchen zeigen:

Figur 1    eine schematische Darstellung eines erfindungsgemäßen ophthalmochirurgischen Systems mit einer Steuerungsvorrichtung;

Figur 2    eine Querschnittsansicht einer Augenvorderkammer mit einer Augenlinse bei einer ersten Nadelposition zum Zeitpunkt $t_1$;

Figur 3    eine Querschnittsansicht einer Augenvorderkammer mit einer Augenlinse bei der ersten Nadelposition und einer Zeit $t_2$; und

Figur 4    eine Querschnittsansicht einer Augenvorderkammer mit einer Augenlinse bei einer zweiten Nadelposition zu einer Zeit $t_2$.

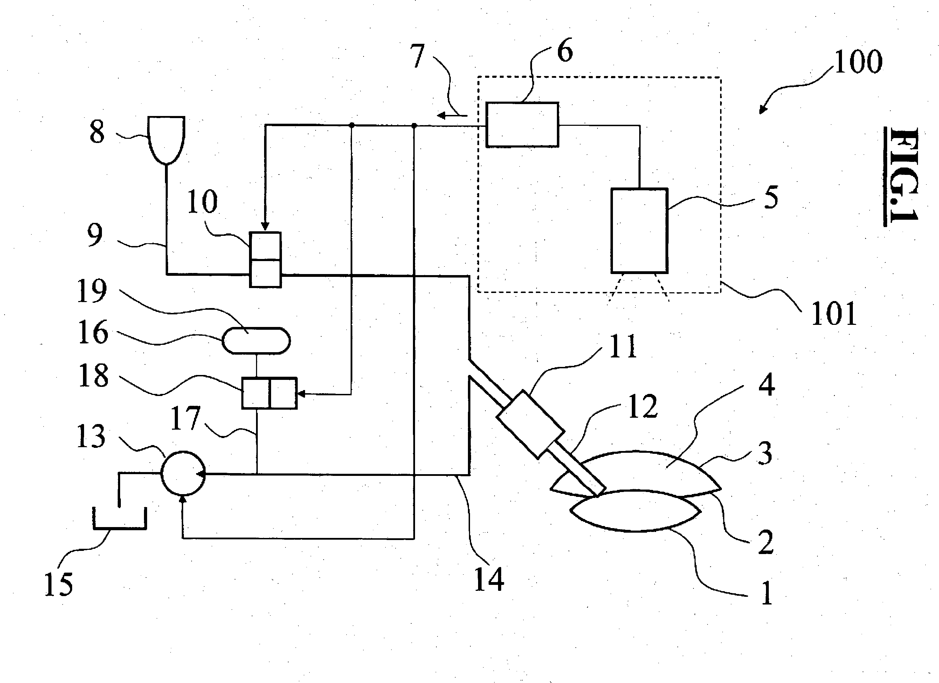

In Figur 1 ist eine schematische Darstellung eines ophthalmochirurgisches Systems 100 mit einer Steuerungsvorrichtung 101 gemäß der Erfindung dargestellt. Oberhalb einer zu behandelnden Augenlinse 1 liegt eine Iris 2 an, welche zusammen mit einer darüber angeordneten Hornhaut 3 die Begrenzung für eine Augenvorderkammer 4 bildet. Eine darüber angeordnete Messvorrichtung 5 ist geeignet, das Volumen der Augenvorderkammer 4 zu einem ersten Zeitpunkt und zu einem zweiten Zeitpunkt zu erfassen, wobei dieser Volumenbetrag zu einer Steuereinheit 6 weitergeleitet wird, welche daraus eine Steuergröße 7 berechnet. Wenn von einem ersten Irrigationsfluidbehälter 8 durch eine Irrigationsleitung 9 und einem Ventil 10 ein Irrigationsfluid zu einem Phakohandstück 11 mit einer schwingenden Nadel 12 geleitet wird, kann die Steuergröße 7 das Ventil 10 entsprechend ansteuern, um einen weiteren Zufluss des Irrigationsfluids vom Irrigationsfluidbehälter 8 zum Handstück 11 zu vermeiden.

Eine Aspirationspumpe 13 saugt mittels einer Aspirationsleitung 14 ein Fluid und entsprechend kleine Partikel aus der Augenvorderkammer 4 zu einem Aspirationsbehälter 15. Die Steuergröße 7 kann hier zum Beispiel auf die Aspirationspumpe 13 einwirken, sodass die Aspirationspumpe 13 bei einer Okklusion mit einer geringeren Pumpleistung arbeitet, so dass sich in der Aspirationsleitung 14 kein so starker Unterdruck ausbilden kann.

Falls bei einer Okklusion ein starker Unterdruck in der Aspirationsleitung vorhanden ist und ein Belüften der Aspirationsleitung erfolgen soll, kann das ophthalmochirurgische System 100 auch einen zweiten Irrigationsbehälter 16 aufweisen, von welchem sich Irrigationsfluid 19 durch eine Belüftungsleitung 17 mittels eines Ventils 18 in die Aspirationsleitung 14 einfüllen lässt. Die Steuergröße 7 kann somit auch das Ventil 18 entsprechend ansteuern, so dass ein Unterdruck in der Aspirationsleitung 14 rasch kompensiert werden kann.

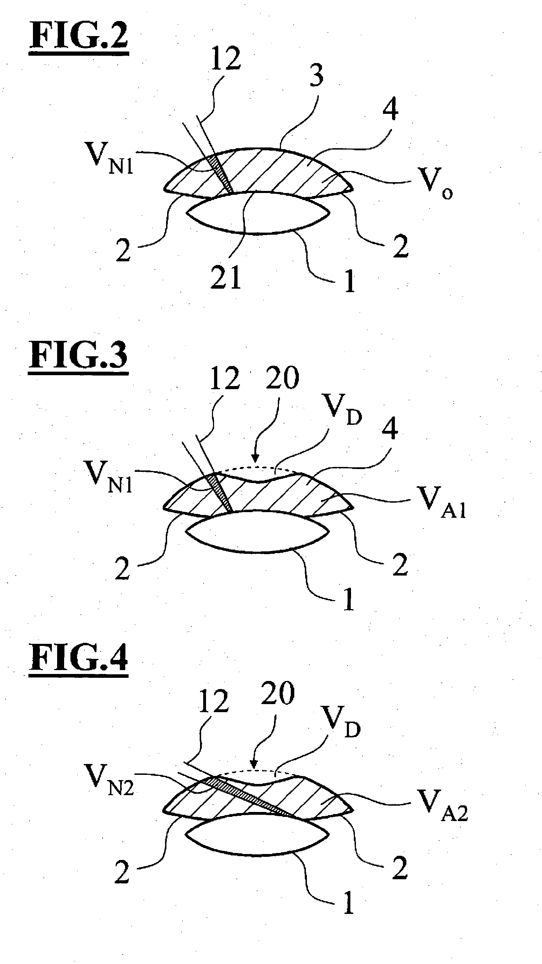

In Figur 2 ist eine Querschnittsansicht einer Augenvorderkammer 4 mit einer zugehörigen Augenlinse 1 dargestellt. Eine Nadel 12 dient dazu, von der Augenlinse 1 kleine Partikel zu erzeugen. Die Augenvorderkammer 4 wird gebildet durch einen oberen Teil 21 eines Linsenkapselsackes, eine Iris 2 und eine Hornhaut 3. Das sich derart ausbildende Volumen der Augenvorderkammer ist in Figur 2 mit $V_0$ bezeichnet. Das Profil der Hornhaut 3 ist konvex dargestellt, wobei sich ein solches Profil bildet, wenn in der Augenvorderkammer 4 noch kein Unterdruck besteht. In dem Bereich der Augenvorderkammer 4 ist eine Nadel 12 eingesetzt, welche bei dieser ersten Nadelposition ein Volumen $V_{N1}$ innerhalb des Volumens $V_0$ der Augenvorderkammer benötigt. Das für eine Druckänderung in einer Augenvorderkammer zum Zeitpunkt $t_1$ verfügbare Volumen $V_{A1}(t_1)$ berechnet sich somit wie folgt:

$$V_{A1}(t_1) = V_0 - V_{N1}.$$

Wenn sich bei dieser ersten Nadelposition ein Unterdruck in der Augenvorderkammer 4 ausbildet, verformt sich die Hornhaut 3 in der Weise, dass sie ihre durchgehend konvexe Form verliert und sich zum Beispiel in der Mitte einwölbt, siehe Bezugszeichen 20 in Fig. 3. Dieser durch eine Druckänderung entstehende Volumenanteil der Augenvorderkammer 4 ist in Figur 3 mit $V_D$ bezeichnet. Damit berechnet sich das Volumen $V_{A1}$ der Augenvorderkammer zu einem Zeitpunkt $t_2$ bei einem sich ausbildenden Unterdruck in der Augenvorderkammer wie folgt:

$$V_{A1}(t_2) = V_0 - V_{N1} - V_D(t_2).$$

In Figur 4 ist eine Nadel in einer zweiten Nadelposition dargestellt. Im Unterschied zu Figur 3 ist die Nadel 12 relativ weit in die Augenvorderkammer 4 hinein geschoben, so dass sie ein Volumen $V_{N2}$ innerhalb des

Volumens $V_0$ der Augenvorderkammer beansprucht. Das Volumen $VA_2$ der Augenvorderkammer berechnet sich zu einem Zeitpunkt $t_2$ dann wie folgt:

$$V_{A2}(t_2) = V_0 - V_{N2} - V_D(t_2).$$

Wenn die Messvorrichtung somit auch den Teil der Nadel erfassen kann, der sich innerhalb der Augenvorderkammer 4 befindet, kann das verfügbare Augenvolumen genauer berechnet werden.

Ist eine Differenz aus $V_{A1}(t_1)$ zu $V_{A1}(t_2)$ ungleich null, bedeutet dies, dass eine Volumenänderung erfolgt ist, so dass zuvor eine Druckänderung in der Augenvorderkammer vorgelegen haben muss. In Abhängigkeit von dem Differenzbetrag kann anschließend der Druck oder der Volumenstrom in einer Irrigationsleitung 9 und/oder Aspirationsleitung 14 mittels der erfindungsgemäßen Steuerungsvorrichtung gesteuert werden.

**Patentansprüche**

1. Steuerungsvorrichtung (101) für ein ophthalmochirurgisches System (100), aufweisend:

   - eine Messvorrichtung (5), mit welcher sich das Volumen einer Augenvorderkammer (4) eines während einer Phakoemulsifikation zu behandelnden Auges (1) zu einem ersten Zeitpunkt ($t_1$) und zu einem zweiten Zeitpunkt ($t_2$) ermitteln lässt,
   - eine Steuereinheit (6), welche in Abhängigkeit von dem ermittelten Volumen zum ersten Zeitpunkt ($t_1$) und zum zweiten Zeitpunkt ($t_2$) eine Steuergröße (7) berechnet, mit der sich ein Druck oder ein Volumenstrom in einer Irrigationsleitung (9) und/oder einer Aspirationsleitung (14) des ophthalmochirurgischen Systems (100) steuern lässt.

2. Steuerungsvorrichtung (101) nach Anspruch 1, wobei die Messvorrichtung (5) ein OCT-System zur Vermessung eines dreidimensionalen Objektbereiches aufweist.

3. Steuerungsvorrichtung (101) nach Anspruch 1, wobei sich mittels der Messvorrichtung (5) das Volumen einer Nadel (12) eines Phakohandstückes (11) innerhalb der Augenvorderkammer (4) ermitteln lässt.

4. Steuerungsvorrichtung (101) nach einem der vorherigen Ansprüche, wobei die Messvorrichtung (5) geeignet ist, die Topographie einer Hornhaut (3) des mittels Phakoemulsifikation zu behandelnden Auges (1) zu vermessen.

**5.** Steuerungsvorrichtung (101) nach Anspruch 4, wobei die Messvorrichtung (5) ein Keratometer aufweist.

**6.** Steuerungsvorrichtung (101) nach einem der vorhergehenden Ansprüche, wobei sich mittels der Steuergröße eine Pumpleistung einer Aspirationspumpe, (13) ein Volumenstrom eines Fluids (19) zur Belüftung der Aspirationsleitung (14) oder ein Ventil (10) in der Irrigationsleitung (9) steuern lässt.

**7.** Ophthalmochirurgisches System mit einer Steuerungsvorrichtung nach einem der Ansprüche 1 bis 6.

**Claims**

**1.** Control device (101) for an ophthalmic surgical system (100), comprising:

- a measuring device (5) by means of which it is possible, at a first time ($t_1$) and a second time ($t_2$), to establish the volume of an anterior chamber of the eye (4) of an eye (1) to be treated by phacoemulsification,
- a control unit (6) which, dependent on the established volume at the first time ($t_1$) and at the second time ($t_2$), calculates a control variable (7) by means of which it is possible to control a pressure or a volumetric flow rate in an irrigation line (9) and/or an aspiration line (14) of the ophthalmic surgical system (100).

**2.** Control device (101) according to Claim 1, wherein the measuring device (5) has an OCT system for measuring a three-dimensional object region.

**3.** Control device (101) according to Claim 1, wherein the measuring device (5) can be used to establish the volume of a needle (12) of a phacoemulsification handpiece (11) within the anterior chamber of the eye (4).

**4.** Control device (101) according to one of the preceding claims, wherein the measuring device (5) is suitable for measuring the topography of a cornea (3) of the eye (1) to be treated by phacoemulsification.

**5.** Control device (101) according to Claim 4, wherein the measuring device (5) has a keratometer.

**6.** Control device (101) according to one of the preceding claims, wherein the control variable can be used to control a pump power of an aspiration pump (13), a volumetric flow rate of a fluid (19) for aerating the aspiration line (14) or a valve (10) in the irrigation line (9).

**7.** Ophthalmic surgical system having a control device according to one of Claims 1 to 6.

**Revendications**

**1.** Dispositif de commande (101) pour système ophtalmo-chirurgical (100), le dispositif présentant :

un dispositif de mesure (5) qui permet de déterminer pendant une phaco-émulsification le volume de la chambre oculaire avant (4) de l'oeil (1) à traiter à un premier instant ($t_1$) et à un deuxième instant ($t_2$),
une unité de commande (6) qui, en fonction du volume ainsi déterminé au premier instant ($t_1$) et au deuxième instant ($t_2$), calcule une grandeur de commande (7) qui permet de commander la pression ou un écoulement volumique dans un conduit d'irrigation (9) et/ou un conduit d'aspiration (14) du système ophtalmo-chirurgical (100).

**2.** Dispositif de commande (101) selon la revendication 1, dans lequel le dispositif de mesure (5) présente un système OCT de mesure tridimensionnelle d'une partie d'un objet.

**3.** Dispositif de commande (101) selon la revendication 1, dans lequel le volume d'une aiguille (12) d'une pièce manuelle phacologique (11) placée à l'intérieur de la chambre oculaire avant (4) peut être déterminé au moyen du dispositif de mesure (5).

**4.** Dispositif de commande (101) selon l'une des revendications précédentes, dans lequel le dispositif de mesure (5) convient pour mesurer la topographie de la cornée (3) de l'oeil (1) à traiter par phaco-émulsification.

**5.** Dispositif de commande (101) selon la revendication 4, dans lequel le dispositif de mesure (5) présente un kératomètre.

**6.** Dispositif de commande (101) selon l'une des revendications précédentes, dans lequel la capacité d'une pompe d'aspiration (13), le débit volumique d'un fluide (19) de ventilation du conduit d'aspiration (14) ou une soupape (10) prévue dans le conduit d'irrigation (9) peuvent être commandés au moyen de la grandeur de commande.

**7.** Système ophtalmo-chirurgical doté d'un dispositif de commande selon l'une des revendications 1 à 6.

FIG.1

EP 2 621 426 B1

## FIG.2

$V_{N1}$  12  3  4  $V_o$

2  21  1  2

## FIG.3

$V_{N1}$  12  20  $V_D$  4  $V_{A1}$

2  2  1

## FIG.4

$V_{N2}$  12  20  $V_D$  $V_{A2}$

2  2  1